Europäisches Patentamt

(19) European Patent Office

Office européen des brevets

(11) **EP 0 531 105 B1**

(12) **EUROPEAN PATENT SPECIFICATION**

(45) Date of publication and mention
of the grant of the patent:
**16.04.1997 Bulletin 1997/16**

(51) Int. Cl.[6]: **A61K 31/27**

(21) Application number: **92307952.9**

(22) Date of filing: **02.09.1992**

(54) **Use of felbamate for the manufacture of a medicament for the treatment of neuropsychopharmacological disorders**

Verwendung von Felbamate zur Herstellung eines Arzneimittels zur Behandlung neuropsychopharmakologischer Störungen

Utilisation de felbamate pour la fabrication d'un médicament pour le traitement de maladies neuropsychopharmacologiques

(84) Designated Contracting States:
**AT BE CH DE DK ES FR GB GR IE IT LI LU MC NL PT SE**

(30) Priority: **03.09.1991 US 753748**

(43) Date of publication of application:
**10.03.1993 Bulletin 1993/10**

(73) Proprietor: **CARTER-WALLACE, INC.**
**New York, N.Y. 10105 (US)**

(72) Inventor: **Sofia, Robert Duane**
**Willingboro, New Jersey 08846 (US)**

(74) Representative: **W.P. Thompson & Co.**
**Coopers Building,**
**Church Street**
**Liverpool L1 3AB (GB)**

(56) References cited:
**EP-A- 0 270 290**          **US-A- 4 978 680**

- **EPILEPSIA, vol. 33, no. 3, 1992, pages 564-572; H. STEVE WHITE ET AL: 'A neuropharmacological evaluation of Felbamate as a novel anticonvulsant'**
- **EPILEPSIA, vol. 27, no. 1, 1986, pages 27-34; EWART A. SWINYARD: 'Comparative anticonvulsant activity and neurotoxicity of felbamate and four prototype antiepileptic drugs in mice and rats'**
- **NEUROLOGY, vol. 40, no. 4/1, 1990, page 193; R.A.WALLIS ET AL.: 'Felbamate is a potent anti-hypoxic agent in the hippocampal slice'**
- **DRUGS OF THE FUTURE, vol. 11, no. 11, 1986, pages 931-932; 'felbamate'**
- **ARZNEIMITTELFORSCHUNG/DRUG RESEARCH, vol. 40, no. 1, May 1990, pages 511-514; L.TURSKI: 'N-Methyl-D-aspartat-Rezeptorkomplex'**
- **The Lancet, 20. July 1985, p. 140-143**
- **Antiepileptic Drugs, Raven Press 1989, p. 572-573**
- **British National Formulary, September 1991, p. 171-182**

Remarks:
The file contains technical information submitted after the application was filed and not included in this specification

## Description

The present invention relates to use of 2-phenyl-1,3-propanediol dicarbamate (felbamate) for treating and controlling the symptoms of the neurodegenerative disorders Guam ALS, Parkinsons disease, Alzheimers disease, dementia and lathyrism which are associated with or result from excessive activation of the strychnine-insensitive glycine receptor sites of the N-methyl-D-aspartate receptor complex.

Neurochemical and electrophysiological studies have demonstrated that glycine modulates excitatory neurotransmission in the central nervous system. These actions are mediated through strychnine-insensitive glycine receptors with markedly different structural requirements for ligand binding and regional distribution than strychnine-sensitive sites associated with the role of glycine as an inhibitory neurotransmitter.

Neurotransmissions mediated through the N-methyl-D-aspartate (NMDA) receptor supramolecular complex are believed to be associated with numerous pathologic and physiologic mechanisms which include: kindling development, ischemic neuronal injury, synaptogenesis, spatial learning and long-term potentiation. Regulation of these neuronal mechanisms by NMDA-mediated processes is believed to involve activation of a receptor-gated ion channel.

Evidence now available clearly indicates that the NMDA complex is regulated, at least in part, by the amino acid glycine. Glycine has been shown to increase NMDA evoked currents in various tissues by increasing the opening frequency of the NMDA channel. Thus, NMDA-induced influx and intracellular accumulation of calcium are stimulated by glycine, which interacts with its own distinct site on the receptor. It is believed that the accumulation of intracellular calcium is implicated in the various neuropathologies.

In Arzneimittel Forschung Vol. 40, 1, 1990 pages 511-514 it is stated, on the basis of animal experimentation, that modulators of NMDA - mediated neutrotransmission may have antiepileptic effects.

Up to the present time, all drugs used in the treatment of neuropsychopharmacological disorders and neurodegenerative diseases function as prophylactics against the symptoms of these disorders and diseases as opposed to being curatives.

For example, US-A-4978680 describes the use of felbamate in the prophylactic treatment of epileptic seizures.

In accordance with the present invention, it has been established that felbamate interacts with strychnine-insensitive glycine receptors and that neuroprotective effects of felbamate are at least in part mediated through a strychnine-insensitive glycine receptor mechanism.

Felbamate is a well-known pharmaceutical compound having been described together with methods for its manufacture and use in US-A- 2,884,444; US-A- 4,868,327; US-A- 4,978,680; US-A- 5,055489; US-A- 5,072,056 and US-A- 5,082,861.

It has now been found possible to provide methods and compositions for the treatment and control of the symptoms of the neurodegenerative diseases Guam ALS, Parkinsons disease, Alzheimers disease, dementia and lathyrism.

Accordingly, in the present invention it has been found that felbamate chemically described as 2-phenyl-1,3-propanediol dicarbamate is a compound which has demonstrated superior properties with respect to the treatment of the neurodegenerative diseases, Guam ALS, Parkinson's disease, Alzheimer's disease, dementia and lathyrism.

According to the present invention there is provided the use of the dicarbamate of 2-phenyl-1,3-propanediol for the manufacture of a medicament for treating and controlling the symptoms of, or for the prevention and control of the neurodegenerative disorders Guam ALS, Parkinsons disease, Alzheimers disease, dimentia and lathyrism which are associated with or result from excessive activation of the strychnine-insensitive glycine receptor sites of the N-methyl-D-aspartate receptor complex in human or other warm blooded animal patients.

The compositions of the present invention may take any of a variety of forms although they are intended primarily for oral use and are suitable for forming into pills, capsules and tablets by well-known practices. Such forms may be dosage unit forms. When the active ingredient is in the form of a solid, a typical tablet composition comprises 500 milligrams of 2-phenyl-1,3-propanediol dicarbamate intermixed in a dry pulverulent state with suitable solid carriers and diluents.

Solid carriers and diluents suitable for use include sugars such as, for example, lactose and sucrose; cellulose derivatives such as, for example, carboxymethyl cellulose, ethyl cellulose, methyl cellulose; gelatin including hard and soft gelatin capsules, talc, cornstarch, stearic acid and magnesium stearate.

Solid carriers and diluents suitable for use include sugars such as, for example, lactose and sucrose; cellulose derivatives such as, for example, carboxymethyl cellulose, ethyl cellulose, methyl cellulose; gelatin including hard and soft gelatin capsules, talc, cornstarch, stearic acid and magnesium stearate.

The percentage of 2-phenyl-1,3-propanediol dicarbamate in the compositions may be varied over wide limits and the quantity of medicament furnished by each individual tablet or capsule is relatively unimportant since the indicated total daily dose can be reached by administering either one or a plurality of capsules or tablets.

In general, an effective daily dose of the active ingredient is in the range of from about 100 milligrams to about 5 grams.

Felbamate (2-phenyl-1,3-propanediol dicarbamate) has a very favourable preclinical profile characterized by a substantial margin of safety (protective index 16.9 - 19.1).

The present invention will now be further described with reference to, but is in no manner limited to, the following Example.

In the Example, the ability of felbamate to interact with strychnine-insensitive glycine receptors is demonstrated. More particularly the radio ligand binding to glycine receptors is demonstrated using the antagonist [3H]5,7-dichlorokynurenic acid, commonly referred to as [3H]5,7DCKA which has been reported to bind with high affinity to glycine receptors in rat membranes. This radiolabelled compound is particularly suitable for purposes of demonstrating the present invention because of its high affinity and reliability within and between experiments.

EXAMPLE

Male Sprague-Dawley rats weighing 150-200 g are housed under a 12-hour light/dark cycle with access to food and water ad libitum. Animals are anesthetized with $CO_2$ and sacrificed by decapitation. Brains are immediately removed and forebrains dissected, weighed and placed in 10 volumes (original wet weight volume) of 5 mM Hepes/4.5 mM Tris buffer (HTS; pH 7.8) containing 0.32 M sucrose. Tissue preparation is performed at about 4°C unless otherwise stated. Tissues are homogenized using a Brinkmann Polytron (setting 6, 30 seconds), diluted to 50 volumes in HTS-sucrose, and centrifuged at 1,000 xg for 10 minutes. The resultant pellet (P1) is discarded and supernatant centrifuged at 20,000 xg for 20 minutes. The resultant pellet (P2) is re-suspended in HTS and centrifuged at 8,000 xg for 20 minutes. The supernatant and outer buffy coat (remaining pellet core discarded) is centrifuged at 20,000 xg for 20 minutes. The resultant pellet (P2/P3) is resuspended in HTS containing 1 mM EDTA and centrifuged at 20,000 xg for 20 minutes. The P2/P3 pellet is resuspended in HTS and the "washing" procedure is repeated two more times. The P2/P3 pellet is then re-suspended in 5 volumes of HTS, frozen on dry-ice and stored at -80°C for at least 72 hours prior to binding assay.

At the time of the assay, the appropriate amount of tissue is thawed and re-suspended in 50 mM Hepes-KOH buffer (pH 8.0 at 4°C) and "washed" twice by re-suspension and centrifugation at 20,000 xg for 20 minutes. The binding of [3H]5,7DCKA is performed as described by Baron et al. Eur J. Pharmacol 206: 149-154 (1991). Assays are performed at 4°C using quadruplicate samples in a total volume/tube of 1-ml consisting of: 500 µl membrane suspension (200-300 µg protein/assay tube) in 50 mM Hepes-KOH buffer (pH 8.0 at 4°C), 100 µl [3H]5,7DCKA solution (19-21 nM; specific activity 18.2 Ci/mmol), 100 µl drug or buffer, and 300 µl buffer. Glycine or 7-chlorokynurenic acid (100 uM) is used to define .pl70 nonspecific binding. Felbamate, solutions of 10mM and 100 mM, are dissolved in 40-50% and 100% DMSO, respectively, and serial dilutions are performed. Binding reactions are initiated by adding the tissue homogenate and terminated after a 15-minute incubation period by rapid filtration through Brandel GF/B filters using a Brandel M-24 cell harvester. This filtration is followed by two 5-ml rinses with ice-cold buffer. Radioactivity is monitored in a Beckman LS 5801 liquid scintillation counter.

Inhibition of [3H]5,7DCKA binding by glycine (10 nM-10µM) or felbamate (10 µM-10mM) is performed to assess the potencies of these compounds for strychnine-insensitive glycine receptors. Displacement curves and $IC_{50}$ values were analyzed using Graphpad (ISI Software, Philadelphia, PA). The $K_1$ values were calculated using the equation: $K_1 = IC_{50}/1 + [L]/K_D$, where [L] is the concentration of [3H]5,7DCKA (19-21 nM) used in the assay and $K_D$ is the dissociation constant of [3H]5,7DCKA (69 nM).

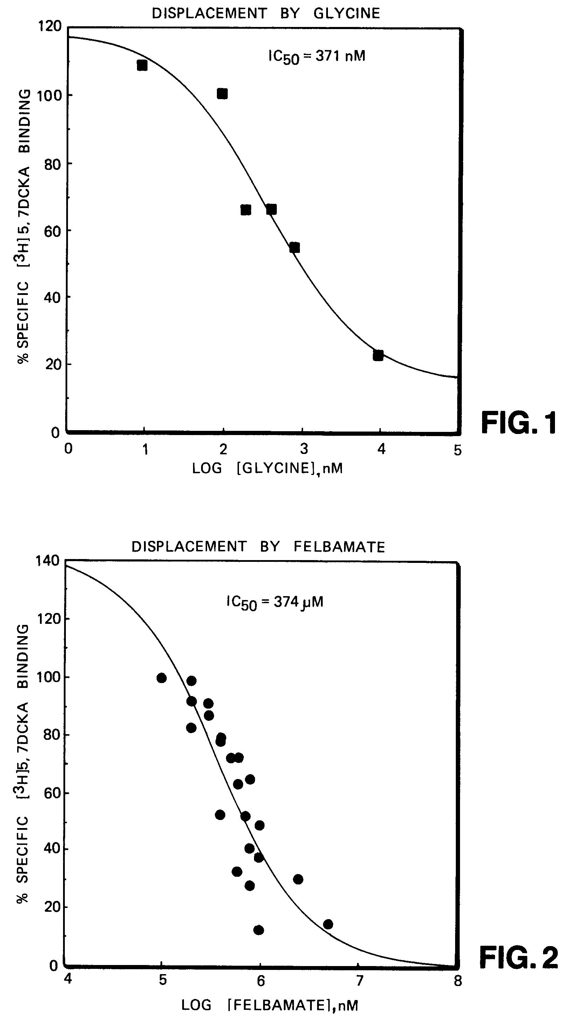

Displacement of [3H]5,7DCKA by glycine (10 nM 10 µM) yielded an $IC_{50}$ of 371 nM (Figure 1). Inhibition of [3H]5,7DCKA by felbamate (10 µM-10mM) resulted in an $IC_{50}$ value of 374 µM (Figure 2). The $IC_{50}$ shown in Figure 2 is generated by combining and then plotting on a single graph the felbamate displacement data from three separate experiments. A Hill slope (-0.93) of these data demonstrated unity and indicated interaction with a single population of sites. The $IC_{50}$ value (mean +/- SEM) from the felbamate displacement experiments presented is calculated to be 477 +/- 49 µM. The $K_1$ estimates for inhibition of [3H]5,7DCKA were: glycine, 284 nM (Figure 1); felbamate, 289 µM; and felbamate (mean +/- SEM), 368 +/- 37 µM (Figure 2).

The $IC_{50}$ value obtained from glycine inhibition of [3H]5,7DCKA (Figure 1) corresponds well with previously reported data. Moreover, results obtained demonstrate tha felbamate interacts with strychnine-insensitive glycine receptors at relatively high concentrations. These conclusions are based on displacement data illustrating that felbamate competitively inhibits [3H]5,7DCKA) binding to rat forebrain membranes (Figure 2).

As previously stated, glycine augments NMDA receptor-mediated membrane depolarization and is required for activation of NMD-gated calcium channels. Furthermore, excessive activation of this "supramolecular complex" (e.g., by excitatory amino acids) has been shown to be associated with seizure disorders, ischemic brain damage and other neuropathologies. Compounds such as felbamate that interact at the strychnine-insensitive glycine modulatory site appear to alter the effects of glycine on the NMDA-gated channels. Thus, the data presented here support a mechanism for neuroprotective effects of felbamate via glycine receptor interaction.

It should be understood that the above Examples is illustrative of the best mode only of the invention herein disclosed.

**Claims**

1.  The use of the dicarbamate of 2-phenyl-1,3-propanediol for the manufacture of a medicament for treating and controlling the symptoms of, or for the prevention and control of the neurodegenerative disorders Guam ALS, Parkinsons disease, Alzheimers disease, dementia and lathyrism which are associated with or result from excessive activation of the strychnine-insensitive glycine receptor sites of the N-methyl-D-aspartate receptor complex in human or other warm blooded animal patients.

2. A use according to claim 1, wherein the medicament is manufactured in the form of a composition comprising also a pharmaceutical carrier, the composition being in dosage unit form.

3. A use according to claim 2, wherein the medicament is manufactured in the form of a gelatin capsule.

4. A use according to claim 2, wherein the medicament is manufactured in the form of a tablet.

5. A use according to any of claims 1 to 4, wherein the neurodegenerative disorder is Alzheimer's disease.

6. A use according to any of claims 1 to 4, wherein the neurodegenerative disorder is Parkinson's disease.

**Patentansprüche**

1. Verwendung des Dicarbamats von 2-Phenyl-1,3-Propandiol für die Herstellung eines Medikamentes zur Behandlung und Kontrolle der Symptome oder zur Prävention und Kontrolle der neurodegenerativen Erkrankungen Guam ALS (auf der Insel Guam gehäuft beobachtete amyotrophische Lateralsklerose), Parkinson-Krankheit, Alzheimer-Krankheit, Demenz und Lathyrismus (Kiechererbsenvergiftung), die mit einer exzessiven Aktivierung der Strychnin-unempfindlichen Glycin-Rezeptororte des N-Methyl-D-Aspartat-Rezeptorkomplexes in menschlichen oder anderen warmblütigen tierischen Patienten einhergehen oder darauf zurückzuführen sind.

2. Verwendung nach Anspruch 1, worin das Medikament in der Form einer Zusammensetzung hergestellt wird, die auch einen pharmazeutischen Träger umfaßt, wobei die Zusammensetzung in der Form einer Dosierungseinheit vorliegt.

3. Verwendung nach Anspruch 2, worin das Medikament in der Form einer Gelatinekapsel hergestellt wird.

4. Verwendung nach Anspruch 2, worin das Medikament in der Form einer Tablette hergestellt wird.

5. Verwendung nach einem der Ansprüche 1 bis 4, worin die neurodegenerative Erkrankung die Alzheimer-Krankheit ist.

6. Verwendung nach einem der Ansprüche 1 bis 4, worin die neurodegenerative Erkrankung die Parkinson-Krankheit ist.

**Revendications**

1. L'utilisation de dicarbamate de 2-phényl-1,3-propanédiol pour la fabrication d'un médicament pour traiter et contrôler les symptômes de, ou pour la prévention et le contrôle des affections neurodégénératives, SLA de Guam (sclérose latérale amyotrophique), maladie de Parkinson, maladie d'Alzheimer, démence et lathyrisme, qui sont associées à ou résultent d'une activation excessive des sites récepteurs de glycocolle insensibles à la strychine du complexe récepteur de D-aspartate de N-méthyle chez les humains ou d'autres patients animaux à sang chaud.

2. Une utilisation selon la revendication 1, dans laquelle le médicament est fabriqué sous la forme d'une composition comprenant également un porteur pharmaceutique, la composition étant sous forme d'unité de dose.

3. Une utilisation selon la revendication 2, dans laquelle le médicament est fabriqué sous la forme d'une gélule.

4. Une utilisation selon la revendication 2, dans laquelle le médicament est fabriqué sous la forme d'un comprimé.

5. Une utilisation selon l'une quelconque des revendications 1 à 4, dans laquelle l'affection neurodégénérative est la maladie d'Alzheimer.

6. Une utilisation selon l'une quelconque des revendications 1 à 4, dans laquelle l'affection neurodégénérative est la maladie de Parkinson.

DISPLACEMENT BY GLYCINE

$IC_{50} = 371$ nM

FIG. 1

DISPLACEMENT BY FELBAMATE

$IC_{50} = 374$ µM

FIG. 2

5